# EUROPEAN PATENT APPLICATION

(11) **EP 4 667 028 A1**
(43) Date of publication of application: **24.12.2025**
(21) Application number: 24756082.4
(22) Date of filing: 04.02.2024
(51) Int. Cl.: A61M 1/16

(54) **LOWER COVER STRUCTURE OF OXYGENATOR, OXYGENATOR HOUSING AND OXYGENATOR**

(30) Priority: 14.02.2023 CN 202310163907; 14.02.2023 CN 202310161579
(71) Applicant: ChinaBridge (Shenzen) Medical Technology Co., Ltd., Shenzhen, Guangdong 518102 (CN)
(72) Inventor: LI, Yijiang, Shenzhen, Guangdong 518102 (CN)
(74) Representative: Pfenning, Meinig & Partner mbB
(86) International application number: PCT/CN2024/075864
(87) International publication number: WO 2024/169708

(57) **Abstract**

The lower cover structure of an oxygenator includes a lower cover body, a liquid inlet tube, and an exhaust tube. A first chamber is provided on the lower cover body. A first isolation ring, a second isolation ring and rib structures are provided in the first chamber. One end of the liquid inlet tube is arranged on the second isolating ring, and the liquid inlet tube is communicated with the channel between the first isolating ring and the second isolating ring. One end of the exhaust tube is arranged on the side wall of the lower cover body, and the exhaust tube is communicated with the channel between the side wall of the lower cover body and the second isolating ring. The liquid inlet tube and the exhaust tube are arranged parallel to each other and on the same side.

## Description

### TECHNICAL FIELD

The present application relates to the technical field of extracorporeal membrane oxygenation of blood, and in particular to an oxygenator lower cover structure, an oxygenator shell, and an oxygenator.

### BACKGROUND

Extracorporeal membrane oxygenation (ECMO) is a method of drawing blood out of the body, oxygenating it through an artificial heart-lung bypass made of special materials, and then injecting it into the patient's arterial or venous system, to partially replace the heart and lungs and maintain the oxygenation and blood supply of human organs and tissues. The oxygenator is an important component in the ECMO system. The oxygenator maintains the blood temperature outside the body and oxygenates the blood. There are mainly three circulation pathways in the oxygenator: the blood path, in which blood enters the oxygenator through a specific channel and is heated by the variable temperature membrane area of the oxygenator to maintain a constant blood temperature; the gas path, in which oxygen penetrates into the blood through the oxygenation membrane filaments in the oxygenation area, and at the same time, carbon dioxide in the venous blood penetrates into the oxygenation membrane filaments and is released from the blood; and the water path, in which a liquid with a constant temperature provides heat energy to the blood through the variable temperature membrane filaments to maintain the temperature of the blood in the extracorporeal circulation pipeline.

The existing oxygenator gas circuit setting is not conducive to improving the oxygenation efficiency. Therefore, a technical issue to be addressed is to improve the oxygenation efficiency of the gas circuit while keeping the blood circuit and water circuit unchanged.

### SUMMARY

In order to solve or alleviate all or part of the above problems, an embodiment provides an oxygenator lower cover structure, an oxygenator housing, and an oxygenator that are conducive to the discharge of blood and gas.

In a first aspect, an embodiment of the present application provides an oxygenator lower cover structure, comprising: a lower cover body, a liquid inlet tube, an exhaust tube and an exhaust port;
The lower cover body is provided with a first chamber, and the first chamber is provided with a first isolation ring, a second isolation ring and a plurality of rib structures;
The second isolation ring is arranged at intervals on the periphery of the first isolation ring, and a plurality of rib structures are arranged at intervals on the periphery of the second isolation ring;
The liquid inlet tube passes through the side wall of the lower cover body and the second isolating ring in sequence from outside to inside, the liquid inlet tube is connected with the passage between the first isolating ring and the second isolating ring, and one end of the liquid inlet tube is arranged on the second isolating ring; the exhaust tube is connected with the passage between the side wall of the lower cover body and the second isolating ring, and one end of the exhaust tube is arranged on the side wall of the lower cover body;
The liquid inlet tube and the exhaust tube are arranged parallel to each other and on the same side, and the exhaust port is arranged in the middle area between the liquid inlet tube and the exhaust tube.

In one embodiment, the diameter of the lower cover body where the exhaust port is located is arranged perpendicular to the liquid inlet tube and the exhaust tube respectively.

In one embodiment, the shortest distance from the exhaust port to the center of the lower cover body is a first distance, the vertical distance between the exhaust port and the tangent line of the lower cover body is a second distance, and the first distance is greater than the second distance.

In one embodiment, the plurality of rib structures are vertically arranged in the first chamber, the plurality of rib structures have the same height, and the plurality of rib structures have a height lower than the side wall height of the lower cover body and the height of the second isolation ring.

In one embodiment, two adjacent rib structures are not connected to each other end to end and are arranged at intervals.

In one embodiment, one end of each of the rib structures is connected to the side wall of the lower cover body, and the other end of each of the rib structures has a preset distance from the side wall of the lower cover body.

In one embodiment, a rib structure is symmetrically distributed on both sides of the liquid inlet tube.

In one embodiment, there is a preset distance between the gas inlet of the exhaust tube and the other end of the adjacent rib structure.

In one embodiment, the liquid inlet tube is arranged along the radial direction of the lower cover body, and the liquid inlet tube and the exhaust tube are arranged on the horizontal plane where the lower cover body is located.

Compared with the prior art, the embodiments provide a lower cover structure of an oxygenator, including: a lower cover body, a liquid inlet tube, an exhaust tube and an exhaust port; a first chamber is arranged on the lower cover body, and a first isolation ring, a second isolation ring and a plurality of rib structures are arranged in the first chamber; the second isolation ring is arranged at intervals on the periphery of the first isolation ring, and a plurality of rib structures are arranged at intervals on the periphery of the second isolation ring; the liquid inlet tube passes through the side wall and the second isolation ring of the lower cover body from the outside to the inside in sequence, the liquid inlet of the liquid inlet tube is connected to the channel between the first isolation ring and the second isolation ring, one end of the liquid inlet tube is arranged on the second isolation ring, the exhaust tube is arranged on the side wall of the lower cover body, the gas inlet of the exhaust tube is connected to the channel between the side wall and the second isolation ring of the lower cover body; the liquid inlet tube and the exhaust tube are arranged in parallel and on the same side, and the exhaust port is arranged in the middle area between the liquid inlet tube and the exhaust tube. The lower cover structure provided by the embodiment of the present application is conducive to the discharge of carbon dioxide after blood oxygenation and is conducive to improving the oxygenation efficiency of the oxygenator.

In a second aspect, an embodiment of the present application further provides an oxygenator housing, comprising an upper cover structure, a housing body, and the oxygenator lower cover structure of the first aspect, wherein the upper cover structure comprises an upper cover body, a blood inlet tube, a liquid discharge tube, and an gas inlet tube;
The upper cover structure and the lower cover structure are arranged at two ends of the shell body;
The upper cover body is provided with a second chamber, and a third isolation ring and a fourth isolation ring are provided in the second chamber; the blood inlet penetrates the third isolation ring and the fourth isolation ring in sequence from the outside to the inside, and the blood inlet is connected with the space surrounded by the fourth isolation ring;
The liquid discharge tube is in communication with the passage between the third isolation ring and the fourth isolation ring, and the gas inlet tube is in communication with the passage between the side wall of the upper cover body and the third isolation ring;
The liquid discharge tube and the liquid inlet tube are parallel to each other and in the same direction, and the gas inlet tube and the exhaust tube are parallel to each other and in the same direction;
The blood inlet tube is respectively arranged vertically with the liquid discharge tube and the gas inlet tube, the liquid discharge tube and the gas inlet tube are arranged parallel to each other and on the same side, and the blood inlet tube, the liquid discharge tube and the gas inlet tube are arranged on the horizontal plane where the upper cover body is located;
A blood outlet tube is arranged on the oxygenator housing at a position corresponding to the blood inlet tube, and the blood outlet tube and the blood inlet tube are arranged on the same side and parallel to each other;
In one embodiment, the blood outlet tube is arranged at the end of the shell body close to the lower cover structure.

In one embodiment, the liquid drainage tube is arranged between the blood inlet tube and the gas inlet tube.

Compared with the prior art, the beneficial effects provided by the second aspect are the same as those of the first aspect and will not be described in detail here.

In a third aspect, an embodiment of the present application further provides an oxygenator, comprising the oxygenator housing described in the second aspect.

Compared with the prior art, the device provided in the third aspect limits the specific regional layout and flow radial direction of the three paths of the gas path, water path or blood path in the oxygenator in the cover body and the shell. This layout and the roughly radial flow of the three paths bring about a better oxygenation effect and reduce the formation of thrombus.

### BRIEF DESCRIPTION OF THE DRAWINGS

FIG. 1 is a schematic front view of an example internal structure of a lower cover structure of an embodiment of a membrane oxygenator.
FIG. 2 is a schematic perspective view of the external structure of the lower cover structure of the embodiment of the membrane oxygenator of FIG. 1.
FIG. 3 is a schematic perspective view of the embodiment of the membrane oxygenator.
FIG. 4 is a schematic perspective view of an example internal structure of an upper cover structure of the embodiment of the membrane oxygenator of FIG. 3.
FIG. 5 is a schematic perspective view of the external structure of the upper cover structure of the embodiment of the membrane oxygenator of FIG. 3.

### DETAILED DESCRIPTION

In order to enable those skilled in the art to better understand the technical solution of the present application, the technical solution in the embodiments is described below in conjunction with the drawings. The described embodiments are only part of the embodiments of the present application, not all of the embodiments. Based on the embodiments in the present application, all other embodiments obtained by ordinary technicians in this field without creative work should fall within the scope of protection of this application.

It should be noted that the terms "first", "second", etc. in the specification and claims of the present application and the above-mentioned drawings are used to distinguish similar objects and are not necessarily used to describe a specific order or sequence. The data used in this way can be interchangeable where appropriate, so that the embodiments of the present application described herein can be implemented in an order other than those illustrated or described herein. In addition, the terms "including" and "having" and any of their variations are intended to cover non-exclusive inclusions, for example, a process, method, system, product or device comprising a series of steps or units is not necessarily limited to those steps or units clearly listed but may include other steps or units that are not clearly listed or inherent to these processes, methods, products or devices.

The oxygenator is one of the devices in the extracorporeal oxygenation system, which can oxygenate venous blood into arterial blood and remove carbon dioxide from the blood to complete the gas exchange of blood outside the body.

Typical existing lower covers of the oxygenator are not conducive to the discharge of the gas after exchange, nor are they conducive to the discharge of condensed water when the warmed gas in the gas path meets the cold gas. Therefore, to solve the problems in the prior art, the present application proposes the following technical solution.

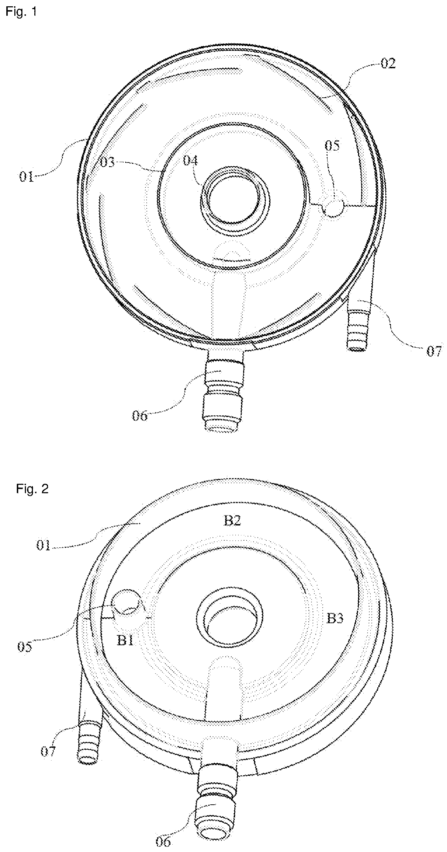

FIG. 1 and FIG. 2 illustrate an embodiment of a lower cover structure of an oxygenator. The lower cover structure includes a lower cover body 01, a liquid inlet tube 06, an exhaust tube 07 and an exhaust port 05.

The lower cover body 01 is provided with a first chamber, which includes a first isolation ring 04, a second isolation ring 03 and a plurality of rib structures 02.

The second isolation ring 03 is arranged at intervals on the periphery of the first isolation ring 04, and the plurality of rib structures 02 are arranged at intervals on the periphery of the second isolation ring 03.

The liquid inlet tube 06 passes through the side wall of the lower cover body 01 and the second isolation ring 03 from the outside to the inside in sequence. The liquid inlet tube 06 is connected (in fluid communication) to the channel between the first isolation ring 04 and the second isolation ring 03. One end of the liquid inlet tube 06 is arranged on the second isolation ring 03. The exhaust tube 07 is arranged on the side wall of the lower cover body 01, and the exhaust tube 07 is connected (in fluid communication) to the channel between the side wall of the lower cover body 01 and the second isolation ring 03.

The liquid inlet tube 06 and the exhaust tube 07 are arranged parallel to each other and on the same side, and the exhaust port 05 is arranged in the middle area between the liquid inlet tube 06 and the exhaust tube 07.

Multiple rib structures 02 are provided to dissipate heat and to guide flow. The plurality of rib structures 02 dissipate heat from the pre-exhausted carbon dioxide and guide the flow of the carbon dioxide to discharged from the exhaust tube 07. In the illustrated embodiment, seven rib structures 02 are provided.

The diameter of the lower cover body 01 where the exhaust port 05 is located is respectively arranged perpendicular to the liquid inlet tube 06 and the exhaust tube 07.

The shortest distance from the exhaust port 05 to the center of the lower cover body 01 is the first distance, the vertical distance between the exhaust port 05 and the tangent line of the lower cover body 01 is the second distance, and the first distance is greater than the second distance.

The position design of the exhaust port 05 is verified by experiments. The positions of the exhaust ports B1/B2/B3 are used as examples of this embodiment. For example, under different oxygen flow rates, the total carbon dioxide removal and total oxygen exchange of the exhaust ports located at B1/B2/B3 are measured respectively. It is found that the gas exchange-related data at the exhaust ports located at B1 are significantly higher than those at the exhaust ports located at B2 and B3. Because condensed water is generated at the outlet of the gas path of the entire oxygenator when it is cooled, the exhaust port 05 can also discharge the condensed water generated at the outlet of the gas path.

The upper cover structure and the lower cover structure of the oxygenator are concentrically arranged, and the cross-sections of the upper cover structure and the lower cover structure are circular. In a particular application, a variable temperature membrane wire is arranged between the first isolation ring 04 and the second isolation ring 03, one end of the variable temperature membrane wire is connected to the space between the third isolation ring 12 and the fourth isolation ring 13 of the upper cover body, and the other end of the variable temperature membrane wire is connected to the space between the first isolation ring 04 and the second isolation ring 03 of the lower cover body. A heat exchange medium, such as water, flows in the annular columnar temperature variable area formed by a plurality of variable temperature membrane wires. The heat exchange medium enters the temperature variable membrane area channel between the first isolation ring 04 and the second isolation ring 03 through the liquid inlet tube 06 and flows out from the discharge tube 10 connected to the space between the third isolation ring 12 and the fourth isolation ring 13 of the upper cover body, so as to heat and keep the blood warm. Similarly, the variable temperature membrane filaments are arranged such that the oxygenation membrane filaments are distributed outside the variable temperature area to form a cylindrical oxygenation area. High-purity oxygen flows from the side wall of the upper cover body 09 and the space between the fourth isolation ring 13 of the upper cover body, through the oxygenation membrane filaments, through the side wall of the lower cover body 01 and the space between the second isolation ring 03 of the lower cover body, and then is discharged through the exhaust tube 07.

After the blood flows from the inlet blood vessel 08 into the area inside the third isolation ring 12 of the upper cover body 09, there may be a shunt structure to disperse the blood flow in the center of the shell body, inside the first isolation ring 04 of the lower cover body 01, and in the central area inside the third isolation ring 12 of the upper cover body 09. As described in Chinese patent application number CN115554505A, the blood is dispersed to the surroundings through the shunt structure, and the blood flows through the temperature-variable membrane filament area and the oxygenation membrane filament area in turn, and finally flows out from the outlet blood vessel on the shell body. Adding a shunt structure has the advantages of better blood dispersion and reducing the pre-filling volume of the oxygenator. For example, a cavity-type shunt structure of Chinese patent application number CN115554505A can also facilitate the fixation of the oxygenator.

The plurality of the rib structures 02 are vertically arranged in the first chamber, the rib structure 02 is a long strip structure, the heights of the plurality of the rib structures 02 are the same, and the plurality of the rib structures 02 are all lower than the side wall height of the lower cover body 01 and the height of the second isolation ring 03. In the embodiment, the plurality of rib structures 02 are vertically arranged in the first chamber because this can increase the contact area between the carbon dioxide gas discharged after blood oxygenation and the rib structure 02, which is beneficial to the flow-guiding effect of the plurality of rib structures 02. The rib structure 02 can increase and make even the flow of the carbon dioxide to the exhaust tube 07 and the exhaust port 05. This "steady flow" effect is more conducive to the flow of the oxygen in the oxygenator, making the oxygenation of the oxygenator more efficient.

In some embodiments, two adjacent rib structures 02 are not connected end to end and are spaced apart from each other, which is beneficial to guide and discharge the oxygenated gas.

In some embodiments, one end of each of the rib structures 02 is connected to the side wall of the lower cover body 01, and a preset distance is provided between the other end of each of the rib structures 02 and the side wall of the lower cover body 01 so that the gas can form a rotating flow along the rib direction and be discharged to the outlet smoothly and quickly and reduce the retention of gas after exchange.

In some embodiments, a rib structure 02 is symmetrically distributed on both sides of the liquid inlet tube 06, which is beneficial to the gas diversion on both sides of the liquid inlet tube 06.

In one embodiment, a preset distance is provided between the exhaust tube 07 and the other end of the adjacent rib structure 02, the liquid inlet tube 06 and the exhaust tube 07 are parallel to each other and are arranged on the same side, and a preset distance is provided between the exhaust tube 07 and the other end of the adjacent rib structure 02 to efficiently facilitate exhaust.

The liquid inlet tube 06 is arranged radially along the lower cover body 01, and the liquid inlet tube 06 and exhaust tube 07 are parallel to each other and arrand on the same the same side, which is beneficial to smoothly guiding the carbon dioxide produced by the oxygenator to the exhaust port 05.

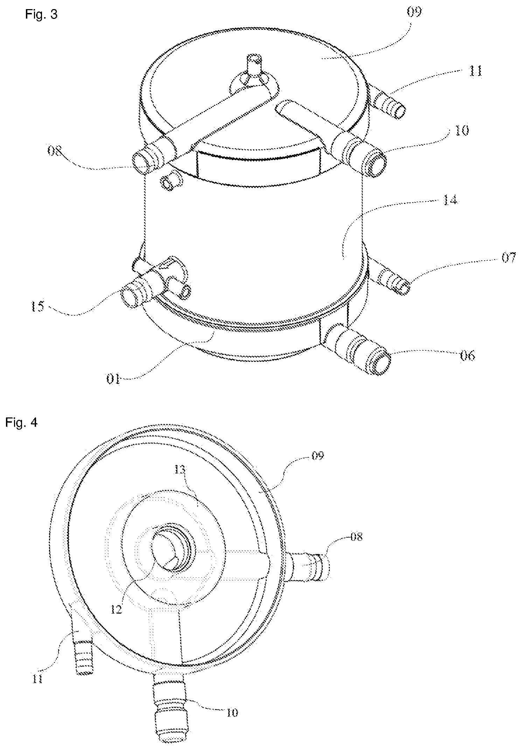

In a second aspect, FIG. 3 illustrates an oxygenator housing comprising the oxygenator lower cover structure, upper cover structure and oxygenator housing 14 described in the first aspect, wherein the upper cover structure and the lower cover structure are arranged at both ends of the housing body 14.

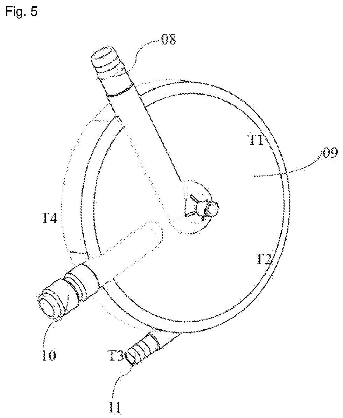

As shown in FIG. 4 and FIG. 5, the upper cover structure includes an upper cover body 09, a blood inlet tube 08, a liquid discharge tube 10, and an gas inlet tube 11.

The upper cover body 09 is provided with a second chamber, in which a third isolation ring 12 and a fourth isolation ring 13 are provided. The blood inlet tube 08 passes through the third isolation ring 12 and the fourth isolation ring 13 from the outside to the inside, and the blood inlet tube 08 is connected (in fluid communication) with the space surrounded by the fourth isolation ring 13;

The drain tube 10 is connected (in fluid communication) with the channel between the third isolation ring 12 and the fourth isolation ring 13. The channel between the third isolation ring 12 and the fourth isolation ring 13 is a channel for the circulation of heat exchange medium, and the heat exchange medium in one example is water. The side wall of the upper cover body 09 and the third isolation ring 12 are connected to form a channel for the circulation of gas, and the gas in one example is oxygen. The space enclosed by the blood inlet tube 08 and the fourth isolation ring 13 is a channel for the circulation of blood.

The liquid discharge tube 10 and the liquid inlet tube 06 are parallel to each other and are in the same direction, and the gas inlet tube 11 and the gas exhaust tube 07 are parallel to each other and are in the same direction.

The blood inlet tube 08 is arranged perpendicularly to the liquid discharge tube 10 and the gas inlet tube 11 respectively. The liquid discharge tube 10 and the gas inlet tube 11 are arranged parallel to each other and on the same side. The blood inlet tube 08, the liquid discharge tube 10 and the gas inlet tube 07 are arranged on the horizontal plane where the upper cover body 09 is located. A blood outlet tube 15 is arranged on the oxygenator housing 14 at a position corresponding to the blood inlet tube 08. The blood outlet tube 15 and the blood inlet tube 08 are arranged on the same side and parallel to each other.

In one embodiment, the blood outlet tube 08 is arranged at the end of the shell body 14 close to the lower cover structure.

In one embodiment, the liquid drain tube 10 is arranged between the blood inlet tube 08 and the gas inlet tube 11.

The arrangement positions of the liquid discharge tube 10 and the gas inlet tube 11 of the upper cover structure are coordinated with the exhaust tube 07 and the liquid inlet tube 06 of the lower cover structure of the oxygenator, which is beneficial to improving the oxygenation efficiency of the blood by the oxygenator. After testing, it was found that the blood oxygenation efficiency of the gas inlet tube 11 arranged at the position T3 is better than that of the gas inlet tube 11 arranged at the positions T1, T2 and T4.

Compared with the prior art, the beneficial effects of the technical solution provided by the second aspect are the same as those of the first aspect and will not be repeated here.

In a third aspect, an embodiment of the present application further provides an oxygenator, comprising the oxygenator housing described in the second aspect.

Compared with the prior art, the beneficial effects of the device provided in the third aspect are the same as those of the first aspect and will not be repeated here.

In a third aspect, an embodiment of the present application further provides an oxygenator, comprising the oxygenator housing described in the second aspect.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. An oxygenator lower cover structure, **characterized in that** it includes: a lower cover body, a liquid inlet pipe, an exhaust pipe and an exhaust port;
A first chamber is provided on the lower cover body, and a first isolation ring, a second isolation ring and a plurality of rib structures are provided in the first chamber;
The second isolation ring is arranged at intervals on the periphery of the first isolation ring, and a plurality of rib structures are arranged on the periphery of the second isolation ring at intervals;
The liquid inlet pipe passes through the side wall of the lower cover body and the second isolation ring in sequence from the outside to the inside. The liquid inlet pipe is connected to the channel between the first isolation ring and the second isolation ring. One end of the liquid inlet pipe is arranged on the second isolation ring, and one end of the exhaust pipe is arranged on the side wall of the lower cover body. The exhaust pipe is connected to the side wall of the lower cover body and the second isolation ring. Channel connections between rings;
The liquid inlet pipe and the exhaust pipe are arranged parallel to each other and on the same side, and the exhaust port is arranged in the middle area of the liquid inlet pipe and the exhaust pipe.

2. An oxygenator lower cover structure as claimed in claim 1, **characterized in that** the diameter of the lower cover body where the exhaust port is located is arranged perpendicularly to the liquid inlet pipe and the exhaust pipe respectively.

3. The oxygenator lower cover structure according to claim 1, **characterized in that** the vertical distance between the straight line where the exhaust port is located and the liquid inlet pipe is greater than the straight line where the exhaust port is located. The vertical distance from the exhaust pipe, the straight line where the exhaust port is located, the liquid inlet pipe and the exhaust pipe are arranged parallel to each other.

4. The oxygenator lower cover structure according to claim 1, **characterized in that** a plurality of rib structures are vertically arranged in the first chamber, and the heights of the plurality of rib structures are the same. And the plurality of rib structures are lower than the side wall height of the lower cover body and the height of the second isolation ring.

5. The oxygenator lower cover structure according to claim 1, **characterized in that** two adjacent rib structures are not connected to each other at the beginning and end and are arranged at intervals.

6. An oxygenator lower cover structure according to claim 1, **characterized in that** one end of each rib structure is connected to the side wall of the lower cover body, and the other end of each rib structure is connected to the side wall of the lower cover body. There is a preset distance between the side walls of the lower cover body.

7. The oxygenator lower cover structure according to claim 1, **characterized in that** a rib structure is symmetrically distributed on both sides of the liquid inlet pipe.

8. The oxygenator lower cover structure according to claim 1, **characterized in that** there is a preset distance between the air inlet of the exhaust pipe and the other end of the adjacent rib structure.

9. The oxygenator lower cover structure according to claim 1, **characterized in that** the liquid inlet pipe is arranged along the radial direction of the lower cover body, and the liquid inlet pipe and the exhaust pipe are arranged On the horizontal plane where the lower cover body is located.

10. An oxygenator, **characterized by** comprising an upper cover structure and an oxygenator lower cover structure as claimed in any one of claims 1 to 9, the upper cover structure comprising an upper cover body, an inlet blood vessel, Drainage and intake pipes;
The upper cover body is provided with a second chamber, and a third isolation ring and a fourth isolation ring are provided in the second chamber; the inlet blood vessel penetrates the third isolation ring and the fourth isolation ring in sequence from outside to inside. A fourth isolation ring, the inlet blood vessel is connected to the space enclosed by the fourth isolation ring;
The drain pipe is connected to the channel between the third isolation ring and the fourth isolation ring, and the air inlet pipe is connected to the channel between the side wall of the upper cover body and the third isolation ring;
The liquid discharge pipe and the liquid inlet pipe are parallel to each other and in the same direction, and the air inlet pipe and the exhaust pipe are parallel to each other and in the same direction;
The inlet blood vessel is arranged perpendicularly to the liquid discharge pipe and the air inlet pipe respectively. The liquid discharge pipe and the air inlet pipe are arranged parallel to each other and on the same side. The inlet blood vessel, the liquid discharge pipe and the air inlet pipe are arranged on the horizontal plane where the upper cover body is located. superior.

11. An oxygenator according to claim 10, **characterized in that** the drain pipe is provided between the inlet blood vessel and the air inlet pipe.

12. An oxygenator housing according to claim 10, **characterized in that** the liquid drain pipe is arranged between the blood inlet pipe and the gas inlet pipe.

13. An oxygenator, **characterized by** comprising the oxygenator according to any of claims 10 to 12.
